# EUROPEAN PATENT APPLICATION

(11) **EP 4 249 590 A1**
(43) Date of publication of application: **27.09.2023**
(21) Application number: 21894756.2
(22) Date of filing: 19.11.2021
(51) Int. Cl.: C12N 5/10, C12N 1/15, C12N 1/19, C12N 1/21, C12N 15/31, C12N 15/55, C12N 15/63, C12N 9/14

(54) **THERMOTOLERANT PROTEIN GLUTAMINASE**

(30) Priority: 20.11.2020 JP 2020193671
(71) Applicant: Amano Enzyme Inc., Nagoya-shi Aichi 460-8630 (JP)
(72) Inventor: MATSUOKA, Tomohiro, Kakamigahara-shi, Gifu 509-0109 (JP); YACHI, Hiroyuki, Kakamigahara-shi, Gifu 509-0109 (JP); SENDA, Daiki, Kakamigahara-shi, Gifu 509-0109 (JP); YUKI, Kensuke, Kakamigahara-shi, Gifu 509-0109 (JP)
(74) Representative: Müller-Boré & Partner Patentanwälte PartG mbB
(86) International application number: PCT/JP2021/042625
(87) International publication number: WO 2022/107885

(57) **Abstract**

Disclosed is a protein glutaminase having improved thermotolerance. A protein glutaminase comprising a polypeptide indicated in any among (1) to (3) has improved thermotolerance. (1) A polypeptide comprising a SEQ ID NO: 1 or 2 amino acid sequence; (2) a polypeptide wherein one or more amino acid residues are substituted, added, inserted or lost in the amino acid sequence indicated by SEQ ID NO: 1 or 2 and exhibiting the same thermotolerance as the amino acid sequence indicated by SEQ ID NO: 1 or 2: and (3) a polypeptide having at least 76% sequence identity with the amino acid sequence indicated by SEQ ID NO: 1 or 2 and having similar thermotolerance to a polypeptide comprising the amino acid sequence indicated by SEQ ID NO: 1 or 2.

## Description

### TECHNICAL FIELD

The present invention relates to a thermostable protein-glutaminase. More specifically, the present invention relates to a thermostable protein-glutaminase, a DNA encoding the thermostable protein-glutaminase, a recombinant vector, a transformant, an enzyme preparation, a method for producing the thermostable protein-glutaminase, and a use of the thermostable protein-glutaminase.

### BACKGROUND ART

Protein-glutaminases are enzymes that deamidate a γ-amide group and a β-amide group of a glutamine residue and an asparagine residue in a protein. Specifically, a protein-glutaminase derived from Chryseobacterium gleum JCM2410 strain and a protein-glutaminase derived from Chryseobacterium proteolyticum 9670 strain (Patent Document 1) are known.

A treatment of a protein with a protein-glutaminase generates a carboxyl group, and therefore is considered to contribute to an increase in the solubility and the water dispersibility of the protein due to an increase in the negative charge of the protein, and to an improvement in the emulsifying power, the emulsion stability, and the like of the protein due to a change in the higher order structure of the protein.

Therefore, protein-glutaminases are used in techniques of modifying functional properties of various proteins. For example, Patent Document 2 discloses a method for modifying a protein by making a protein-glutaminase and a transglutaminase act on the protein, and describes that a treatment with a protein-glutaminase and a transglutaminase improved the smoothness of a produced food in production of yogurt using milk, production of bean curd using soy milk, and production of noodles using wheat. Patent Document 3 discloses a method for producing a processed meat food using arginine or its salt and a protein-glutaminase, and describes that a treatment of a raw meat with an arginine or its salt and a protein-glutaminase improved the texture of an obtained processed food in production of sausage, ham, a hamburger, deep-fried chicken, a pork cutlet, barbecued pork, and the like.

### PRIOR ART DOCUMENTS

### PATENT DOCUMENTS

Patent Document 1: WO 2010/029685 A
Patent Document 2: WO 2009/113628 A
Patent Document 3: WO 2012/060409 A

### SUMMARY OF THE INVENTION

### PROBLEMS TO BE SOLVED BY THE INVENTION

Effects of denaturing a protein by a protein-glutaminase are recognized as causes of various preferable functional properties in protein materials such as foods, and protein-glutaminases are considered to be widely used for food processing and the like in the future. Meanwhile, conventional protein-glutaminases have specific thermal properties, and therefore temperature conditions in protein processing are inevitably limited according to the thermal properties of protein-glutaminases. Therefore, in order to expand the use of protein-glutaminases, a further improvement in the thermostability is awaited.

Therefore, an object of the present invention is to provide a protein-glutaminase having improved thermostability.

### MEANS FOR SOLVING THE PROBLEM

The present inventors have found a protein-glutaminase having improved thermostability by screening from a nonpublic strain library possessed by the applicant. The present invention has been completed on the basis of this finding. That is, the present invention provides the invention of the aspects described below.

Item 1. A protein-glutaminase including a polypeptide shown in any one of the following (1) to (3):
(1) a polypeptide having an amino acid sequence shown in SEQ ID NO: 1 or 2;
(2) a polypeptide having an amino acid sequence obtained by substituting, adding, inserting, or deleting one or more amino acid residues in an amino acid sequence shown in SEQ ID NO: 1 or 2, the polypeptide having thermostability equivalent to thermostability of a polypeptide having an amino acid sequence shown in SEQ ID NO: 1 or 2; and
(3) a polypeptide having an amino acid sequence having a sequence identity to an amino acid sequence shown in SEQ ID NO: 1 or 2 of 76% or more, the polypeptide having thermostability equivalent to thermostability of a polypeptide having an amino acid sequence shown in SEQ ID NO: 1 or 2.

Item 2. A DNA encoding the protein-glutaminase according to the item 1.

Item 3. An expression cassette or a recombinant vector including the DNA according to the item 2.

Item 4. A transformant obtained by transforming a host with the expression cassette or the recombinant vector according to the item 3.

Item 5. A method for producing the protein-glutaminase according to the item 1, the method including a step of culturing the transformant according to the item 4.

Item 6. An enzyme preparation including the protein-glutaminase according to the item 1.

Item 7. A protein modifier including the protein-glutaminase according to the item 1.

Item 8. A method for producing a modified protein material, the method including a step of making the protein-glutaminase according to the item 1 act on a protein material.

Item 9. The method according to the item 8, wherein the protein material is edible.

### ADVANTAGES OF THE INVENTION

According to the present invention, a protein-glutaminase having improved thermostability is provided.

### BRIEF DESCRIPTION OF THE DRAWINGS

Fig. 1 shows an SDS-PAGE image of a purified fraction of a protein-glutaminase derived from Chryseobacterium sp. strain 57594.
Fig. 2 shows an SDS-PAGE image of a purified fraction of a protein-glutaminase derived from Chryseobacterium sp. strain 61798.
Fig. 3 shows test results for temperature stability of protein-glutaminases derived from Chryseobacterium sp. strain 57594 and strain 61798.
Fig. 4 shows test results for optimal temperature of protein-glutaminases derived from Chryseobacterium sp. strain 57594 and strain 61798.
Fig. 5 shows test results for pH stability of protein-glutaminases derived from Chryseobacterium sp. strain 57594 and strain 61798.
Fig. 6 shows test results for optimal pH of protein-glutaminases derived from Chryseobacterium sp. strain 57594 and strain 61798.

### EMBODIMENTS OF THE INVENTION

Hereinafter, the present invention will be described in detail. Except for in a sequence listing, 20 types of amino acid residues in amino acid sequences may be represented by a one-letter abbreviation. That is, glycine (Gly) is represented by G, and alanine (Ala) is A, valine (Val) is V, leucine (Leu) is L, isoleucine (Ile) is I, phenylalanine (Phe) is F, tyrosine (Tyr) is Y, tryptophan (Trp) is W, serine (Ser) is S, threonine (Thr) is T, cysteine (Cys) is C, methionine (Met) is M, aspartic acid (Asp) is D, glutamic acid (Glu) is E, asparagine (Asn) is N, glutamine (Gln) is Q, lysine (Lys) is K, arginine (Arg) is R, histidine (His) is H, and proline (Pro) is P.

In an amino acid sequence described in the present description, the left end indicates the N-terminal, and the right end indicates the C-terminal.

In the present description, "non-polar amino acids" include alanine, valine, leucine, isoleucine, proline, methionine, phenylalanine, and tryptophan. "Non-charged amino acids" include glycine, serine, threonine, cysteine, tyrosine, asparagine, and glutamine. "Acidic amino acids" include aspartic acid and glutamic acid. "Basic amino acids" include lysine, arginine, and histidine.

In the present description, the term "substitution" refers to not only a case where a substitution of an amino acid residue is artificially introduced, but also a case where a substitution of an amino acid residue is naturally introduced, that is, a case where a different amino acid residue is originally introduced. In the present description, a substitution of an amino acid residue may be an artificial substitution or a natural substitution, but an artificial substitution is preferable.

### 1. Protein-Glutaminase

The protein-glutaminase of the present invention includes a polypeptide shown in any one of the following (1) to (3).

(1) a polypeptide having an amino acid sequence shown in SEQ ID NO: 1 or 2;
(2) a polypeptide having an amino acid sequence obtained by substituting, adding, inserting, or deleting one or more amino acid residues in an amino acid sequence shown in SEQ ID NO: 1 or 2, the polypeptide having thermostability equivalent to thermostability of a polypeptide having an amino acid sequence shown in SEQ ID NO: 1 or 2; and
(3) a polypeptide having an amino acid sequence having a sequence identity to an amino acid sequence shown in SEQ ID NO: 1 or 2 of 76% or more, the polypeptide having thermostability equivalent to thermostability of a polypeptide having an amino acid sequence shown in SEQ ID NO: 1 or 2.

The polypeptides shown in (1) to (3) have protein-glutaminase activity and improved thermostability.

The polypeptide shown in (1) is a protein-glutaminase derived from Chryseobacterium sp., and the polypeptides shown in (2) and (3) are variants of the polypeptide shown in (1). The polypeptides of (1) to (3) include not only polypeptides obtained by an artificial substitution but also polypeptides originally having such an amino acid sequence.

In the polypeptide of (2), the amino acid modification introduced may include only one modification (for example, only a substitution) from among a substitution, an addition, an insertion, and a deletion, or may include two or more modifications (for example, a substitution and an insertion). In the polypeptide of (2), the number of amino acid differences at any site of difference is to be 1 or several, and is, for example, 1 to 80, preferably 1 to 70, 1 to 60, 1 to 50, 1 to 40, or 1 to 30, more preferably 1 to 20, 1 to 10, 1 to 8, 1 to 7, 1 to 6, 1 to 5, or 1 to 4, still more preferably 1 to 3, and particularly preferably 1 or 2, or 1.

In the polypeptide of(3), the sequence identity to an amino acid sequence shown in SEQ ID NO: 1 or 2 is to be 76% or more, and is preferably 80% or more, more preferably 85% or more, still more preferably 90% or more, still even more preferably 95% or more, 96% or more, 97% or more, or 98% or more, and particularly preferably 99% or more.

Here, in the polypeptide of (3), the sequence identity to an amino acid sequence shown in SEQ ID NO: 1 or 2 is calculated by comparison with the amino acid sequence shown in SEQ ID NO: 1 or 2. The term "sequence identity" refers to the value of the identity of amino acid sequences, obtained by bl2seq program (Tatiana A. Tatsusova, Thomas L. Madden, FEMS Microbiol. Lett., Vol. 174, p 247-250, 1999) in BLAST PACKAGE [sgi32 bit edition, Version 2.0.12; available from National Center for Biotechnology Information (NCBI)]. The parameters are to be set to Gap insertion Cost value: 11 and Gap extension Cost value: 1

The amino acids at positions 176 (cysteine), 217 (histidine), and 237 (aspartic acid) in the amino acid sequences shown in SEQ ID NOs: 1 and 2 are considered to be protein-glutaminase activity catalyst residues. Therefore, in the polypeptides of (2) and (3), it is desirable not to introduce a substitution or a deletion at these sites.

In the polypeptides of (2) and (3), in a case where an amino acid substitution is introduced into an amino acid sequence shown in SEQ ID NO: 1 or 2, a preferred aspect of the amino acid substitution introduced is, for example, a conservative substitution. That is, the substitution in the polypeptides of (2) and (3) is, for example, a substitution of a non-polar amino acid with another non-polar amino acid if the amino acid before substitution is a non-polar amino acid. If the amino acid before substitution is a non-charged amino acid, the substitution is a substitution with another non-charged amino acid. If the amino acid before substitution is an acidic amino acid, the substitution is a substitution with another acidic amino acid, and If the amino acid before substitution is a basic amino acid, the substitution is a substitution with another basic amino acid.

In the polypeptides of (2) and (3), the phrase "having thermostability equivalent to thermostability of a polypeptide having an amino acid sequence shown in SEQ ID NO: 1 or 2" means that when the thermostability, that is, the protein-glutaminase relative activity at 65°C is measured, the resulting protein-glutaminase relative activity is equivalent to that of the polypeptide of (1), specifically, the resulting protein-glutaminase relative activity is about 0.8 to 1.2 with respect to a protein-glutaminase relative activity of the polypeptide of (1) of 1. The protein-glutaminase relative activity at 65°C refers to the relative amount (%) of the protein-glutaminase activity of an enzyme exposed to a temperature condition of 65°C when the protein-glutaminase activity of the enzyme exposed to a temperature environment of 4°C or 65°C for 10 minutes and exposed to a temperature condition of 4°C is set to 100%.

### 2. DNA

The DNA of the present invention is a DNA encoding the protein-glutaminase described in "l. Protein-Glutaminase" above (hereinafter, also referred to as "the predetermined protein-glutaminase"). Examples of the DNA of the present invention include a DNA having a base sequence shown in SEQ ID NO: 3 or 4. The base sequence shown in SEQ ID NO: 3 is a gene encoding a polypeptide having the amino acid sequence shown in SEQ ID NO: 1, and the base sequence shown in SEQ ID NO: 4 is a gene encoding a polypeptide having the amino acid sequence shown in SEQ ID NO: 2.

The DNA of the present invention is not limited to the above-described sequences, and examples of the DNA of the present invention include DNAs having a base sequence having a homology with a base sequence shown in SEQ ID NO: 3 or 4 of 76% or more, preferably 80% or more, more preferably 85% or more, even more preferably 90% or more, 91% or more, or 92% or more, still even more preferably 95% or more, and particularly preferably 99% or more as long as the DNAs encode a polypeptide having protein-glutaminase activity.

Here, the "homology" of a DNA is calculated using publicly available or commercially available software with an algorithm for comparison using a reference sequence as a query sequence. Specifically, BLAST, FASTA, GENETYX (available from GENETYX CORPORATION), or the like can be used, and these are to be set to the default parameters and used.

Examples of the DNA of the present invention also include DNAs having a base sequence in which a mutation of a substitution, an addition, an insertion, or a deletion is introduced in several bases in a base sequence described in SEQ ID NO: 3 or 4 corresponding to the substitution, the addition, the insertion, or the deletion in the above-described amino acid sequence, as long as the DNAs encode a polypeptide having protein-glutaminase activity.

Examples of the DNA of the present invention also include DNAs that hybridize under a stringent condition with a DNA having a base sequence complementary with a DNA having a base sequence described in SEQ ID NO: 3 or 4, as long as the DNAs encode a polypeptide having protein-glutaminase activity.

Here, the stringent condition is a condition in which a nylon membrane on which a DNA is immobilized is incubated together with a probe in a solution containing 6 × SSC (1 × SSC is obtained by dissolving 8.76 g of sodium chloride and 4.41 g of sodium citrate in 1 liter of water), 1% SDS, 100 µg/mL salmon sperm DNA, 0.1% bovine serum albumin, 0.1% polyvinylpyrrolidone, and 0.1% Ficoll (in the present description, "%" means "w/v") at 65°C for 20 hours to perform hybridization.

For a detailed procedure of the hybridization method, Molecular Cloning, A Laboratory Manual 2nd ed. (Cold Spring Harbor Laboratory Press (1989)) and the like can be referred to.

Hereinafter, an example of a method of obtaining the DNA of the present invention by hybridization will be described, but the method of obtaining the DNA of the present invention is not limited to the following example.

First, a DNA obtained from an appropriate gene source is connected to a plasmid or a phage vector in accordance with a standard method to prepare a DNA library. This library is introduced into an appropriate host to obtain a transformant, the transformant is cultured on a plate, the grown colony or plaque is transferred to a nitrocellulose or nylon membrane, and after denaturation treatment, the DNA is fixed to the membrane. This membrane is incubated, under the above-described stringent condition, in a solution having the above-described composition and containing a probe labeled in advance with 32P or the like to perform hybridization. As the probe, a polynucleotide can be used that encodes all or a part of an amino acid sequence described in SEQ ID NO: 1 or 2.

After completion of hybridization, the probe non-specifically adsorbed is washed away, and the clone hybridized with the probe is identified by autoradiography or the like. This operation is repeated until the hybridized clone can be isolated. Finally, a gene encoding a protein having a target enzyme activity is selected from the obtained clone. The gene can be isolated with a known polynucleotide extraction method such as an alkaline method.

The DNA of the present invention can also be isolated from a microorganism that produces the predetermined protein-glutaminase. For example, a target DNA can be isolated from the genome of the microorganism by PCR or a hybridization method in which, with a genomic DNA derived from Chryseobacterium sp. as a template, a primer or a probe is used that is designed from known amino acid sequence information in consideration of degeneracy of a gene or designed on the basis of known base sequence information.

Examples of the DNA of the present invention include various types of DNAs derived from codon degeneracy. Various types of DNAs encoding the same amino acid sequence can be easily prepared using a known genetic engineering technique. For example, in production of a protein using genetic engineering, in a case where a codon used in an original gene encoding a target protein has a low usage frequency in a host, the expression level of the protein may be low. In such a case, high expression of the target protein can be achieved by optimizing the codon usage frequency for the host without changing the encoded amino acid sequence.

As an index representing the codon usage frequency, the total of host-optimal codon usage frequencies of codons may be used. The optimal codon is defined as a codon having the highest usage frequency of codons corresponding to the same amino acid. The codon usage frequency is not particularly limited as long as it is optimized for the host, and examples of the optimal codon of Escherichia coli include the following codons. F: phenylalanine (ttt), L: leucine (ctg), I: isoleucine (att), M: methionine (atg), V: valine (gtg), Y: tyrosine (tat), termination codon (taa), H: histidine (cat), Q: glutamine (cag), N: asparagine (aat), K: lysine (aaa), D: aspartic acid (gat), E: glutamic acid (gaa), S: serine (age), P: proline (ccg), T: threonine (acc), A: alanine (gcg), C: cysteine (tgc), W: tryptophan (tgg), R: arginine (cgc), and G: glycine (ggc).

Usable methods of introducing a mutation into a gene to modify an amino acid sequence artificially include known methods such as the Kunkel method and a gapped duplex method and methods using a mutation introduction kit by site-directed mutagenesis, such as QuikChange (trademark) Site-Directed Mutagenesis Kit (Stratagene Corp), GeneArt (trademark) Site-Directed Mutagenesis PLUS System (Invitrogen Corporation), or TaKaRa Site-Directed Mutagenesis System (Mutan-K, Mutan-Super Express Km, PrimeSTAR Mutagenesis Basal Kit, etc.: Takara Bio Inc.).

The base sequence of a DNA can be confirmed by sequencing with a conventional method. For example, the base sequence can be confirmed with a dideoxynucleotide chain termination method (Sanger et al. (1977) Proc. Natl. Acad. Sci. USA 74: 5463) or the like. The sequence can be analyzed using an appropriate DNA sequencer.

Whether the obtained DNA is a DNA encoding a target protein-glutaminase can be confirmed by comparing the determined base sequence with a base sequence described in SEQ ID NO: 3 or 4. Alternatively, the confirmation can be performed by comparing the amino acid sequence estimated from the determined base sequence with an amino acid sequence described in SEQ ID NO: 1 or 2.

### 3. Expression Cassette or Recombinant Vector

The expression cassette or the recombinant vector including a DNA encoding the predetermined protein-glutaminase (hereinafter, also referred to as "the expression cassette of the present invention" or "the recombinant vector of the present invention") can be obtained by linking a promoter and a terminator to the DNA of the present invention, or by inserting the expression cassette of the present invention or the DNA of the present invention into an expression vector.

The expression cassette of the present invention or the recombinant vector of the present invention may further include transcriptional elements such as an enhancer, a CCAAT box, a TATA box, and an SPI site as regulators as necessary in addition to a promoter and a terminator. These regulators are to be operatively linked to the DNA of the present invention. The phrase "to be operatively linked" means that various regulators that regulate the DNA of the present invention are linked to the DNA of the present invention in an operative state in a host cell.

As for the recombinant vector of the present invention, the expression vector is preferably constructed for gene recombination from a phage, plasmid, or virus capable of autonomously replicating in a host. Such an expression vector is publicly known, and examples of the commercially available expression vector include a pQE vector (QIAGEN), pDR540, pRIT2T (GE Healthcare Bio Sciences K.K.), and a pET vector (Merck KGaA). The expression vector is to be used in appropriate combination with a selected host cell. Examples of the combination in the case of using Escherichia coli as a host cell include a combination of a pET vector and a DH5α Escherichia coli strain, a combination of a pET vector and a BL21 (DE3) Escherichia coli strain, and a combination of a pDR540 vector and a JM109 Escherichia coli strain.

### 4. Transformant

The expression cassette or recombinant vector of the present invention is used for transforming a host to obtain a transformant (hereinafter, also referred to as "the transformant of the present invention").

The host used for producing the transformant is not particularly limited as long as a gene can be introduced into the host, the expression cassette or recombinant vector is stable, the host can autonomously replicate, and a character of a gene including the DNA of the present invention can be expressed. Preferable examples of the host include bacteria belonging to the genus Escherichia such as Escherichia coli, bacteria belonging to the genus Bacillus such as Bacillus subtilis, and bacteria belonging to the genus Pseudomonas such as Pseudomonas putida; and yeast. In addition, examples of the host may include animal cells, insect cells, and plants.

The transformant of the present invention can be obtained by introducing the expression cassette of the present invention or the recombinant vector of the present invention into a cell of a host. Where the DNA of the present invention is introduced is not particularly limited as long as a target gene can be expressed, and the DNA may be introduced into a plasmid or a genome. Specific examples of the method of introducing the expression cassette of the present invention or the recombinant vector of the present invention include a recombinant vector method and a genome editing method. The condition for introducing the expression cassette or the recombinant vector into a host is to be appropriately set according to the type of the host, and the like. In the case of using a bacterium as a host, examples of the method include a method in which a competent cell by calcium ion treatment is used, and an electroporation method. In the case of using yeast as a host, examples of the method include an electroporation method, a spheroplast method, and a lithium acetate method. In the case of using an animal as a host, examples of the method include an electroporation method, a calcium phosphate method, and a lipofection method. In the case of using an insect as a host, examples of the method include a calcium phosphate method, a lipofection method, and an electroporation method. In the case of using a plant as a host, examples of the method include an electroporation method, an Agrobacterium method, a particle gun method, and a PEG method.

Whether the expression cassette of the present invention or the recombinant vector of the present invention has been incorporated into a host can be confirmed with a PCR method, a Southern hybridization method, a Northern hybridization method, or the like.

In the case of confirming whether the expression cassette of the present invention or the recombinant vector of the present invention has been incorporated into a host with PCR, for example, the genomic DNA, the expression cassette, or the recombinant vector is to be separated and purified from the transformant.

For example, in the case of using a bacterium as a host, the expression cassette or the recombinant vector is separated and purified from a lysate obtained by lysing the bacterium. As a method of lysis, for example, treatment with a lytic enzyme such as lysozyme is performed using, as necessary, a protease, another enzyme, and a surfactant such as sodium lauryl sulfate (SDS) in combination.

Furthermore, a method of physical crushing such as freeze thaw or French press may be combined. The DNA can be separated and purified from the lysate, for example, by appropriate combination of deproteinization by a phenol treatment and a protease treatment, a ribonuclease treatment, an alcohol precipitation treatment, and a commercially available kit.

The DNA can be cleaved in accordance with a standard method, for example, using a restriction enzyme treatment. As the restriction enzyme, for example, a type II restriction enzyme is used that acts on a specific nucleotide sequence. The DNA and the expression cassette or expression vector are bound, for example, using a DNA ligase.

Thereafter, a primer specific to the DNA of the present invention is designed using the separated and purified DNA as a template, and PCR is performed. The amplification product obtained by PCR is subjected to agarose gel electrophoresis, polyacrylamide gel electrophoresis, capillary electrophoresis, or the like, stained with ethidium bromide, SYBR Green solution, or the like, and then detected as a band to confirm the transformation.

Alternatively, the amplification product can be detected by PCR using a primer labeled in advance with a fluorescent dye or the like. A method may also be used in which the amplification product is bound to a solid phase such as a microplate and confirmed using fluorescence, an enzyme reaction, or the like.

### 5. Method for Producing Protein-Glutaminase

The predetermined protein-glutaminase can be obtained by a method, for producing the predetermined protein-glutaminase, including a step of culturing the transformant of the present invention or a method, for producing the predetermined protein-glutaminase, including a step of culturing a microorganism that produces the predetermined protein-glutaminase.

The culture condition is to be appropriately set in consideration of the nutritional physiological properties of the transformant or the microorganism, and liquid culture is preferable. In the case of industrial production, aeration-agitation culture is preferable.

As the nutrient source of the culture medium, a nutrient source required for growth of the transformant or the microorganism can be used. The carbon source is to be an assimilable carbon compound, and examples of such a compound include glucose, sucrose, lactose, maltose, molasses, and pyruvic acid.

The nitrogen source is to be an assimilable nitrogen compound, and examples of such a compound include peptone, meat extracts, yeast extracts, casein hydrolysates, and soybean cake alkaline extracts.

A nutrient source other than the carbon source and the nitrogen source may be used as necessary, and examples of such a nutrient source include phosphates, carbonates, sulfates, salts of magnesium, calcium, potassium, iron, manganese, zinc, and the like, specific amino acids, and specific vitamins.

The culture temperature can be appropriately set within a range in which the transformant of the present invention or the microorganism can grow and the transformant or the microorganism produces the predetermined protein-glutaminase, and is, for example, about 10 to 40°C, and preferably about 15 to 37°C. Completion of the culture is to be appropriately timed to attain the highest yield of the predetermined protein-glutaminase, and the culture time is usually about 6 to 96 hours or about 12 to 48 hours.

After the culture of the transformant or the microorganism, the culture supernatant or the bacterial cell is collected with a method such as centrifugation of the culture solution, the bacterial cell is treated with a mechanical method in which an ultrasonic wave, French press, or the like is used, or treated with a lytic enzyme such as lysozyme by using, as necessary, an enzyme such as a protease and a surfactant such as sodium lauryl sulfate (SDS) for solubilization to obtain a water-soluble fraction containing the predetermined protein-glutaminase.

Furthermore, selection of an appropriate expression cassette or expression vector and a host also allows secretion of the expressed predetermined protein-glutaminase in the culture solution.

The water-soluble fraction containing the predetermined protein-glutaminase obtained as described above may be directly subjected to a purification treatment, or may be subjected to a purification treatment after concentration of the predetermined protein-glutaminase in the water-soluble fraction.

The concentration can be performed, for example, by vacuum concentration, membrane concentration, a salting-out treatment, or a fractional precipitation method by use of a hydrophilic organic solvent (such as methanol, ethanol, or acetone).

The purification treatment of the predetermined protein-glutaminase can be performed, for example, by appropriately combining methods such as gel filtration, adsorption chromatography, ion exchange chromatography, and affinity chromatography.

The predetermined protein-glutaminase purified as described above can be powdered by freeze-drying, vacuum-drying, spray-drying, or the like as necessary and distributed to the market.

### 6. Enzyme Preparation

The predetermined protein-glutaminase can be provided in a form of an enzyme preparation. Therefore, the present invention also provides an enzyme preparation including the predetermined protein-glutaminase as an active component.

The content of the predetermined protein-glutaminase in the enzyme preparation of the present invention is not particularly limited, and is, for example, 0.1 U/g or more or 1 U/g or more, preferably 10 U/g or more, more preferably 100 U/g or more, still more preferably 250 U/g or more, and particularly preferably 500 U/g or more, 1000 U/g or more, 1500 U/g or more, 2000 U/g or more, or 5000 U/g or more.

The enzyme preparation of the present invention may include another component in addition to the predetermined protein-glutaminase to an extent such that as an effect of the present invention is not affected. Examples of another component include enzymes other than the predetermined protein-glutaminase, additives, and culture residues generated in the above-described method for producing.

Examples of another enzyme include amylases (α-amylases, β-amylases, glucoamylases), glucosidases (α-glucosidases, β-glucosidases), galactosidases (α-galactosidases, β-galactosidases), proteases (acidic proteases, neutral proteases, alkaline proteases), peptidases (leucine peptidases, aminopeptidases), lipases, esterases, cellulases, phosphatases (acidic phosphatases, alkaline phosphatases), nucleases, deaminases, oxidases, dehydrogenases, glutaminases, pectinases, catalases, dextranases, transglutaminases, protein deamidases (other than the above-described active components), and pullulanases. These other enzymes may be included singly or in combination of two or more thereof.

Examples of the additives include excipients, buffers, suspending agents, stabilizers, preservatives, antiseptics, and physiological saline. Examples of the excipients include starch, dextrin, maltose, trehalose, lactose, D-glucose, sorbitol, D-mannitol, sucrose, and glycerol. Examples of the buffers include phosphates, citrates, and acetates. Examples of the stabilizers include propylene glycol and ascorbic acid. Examples of the preservatives include benzoates (alkali metal salts such as potassium salts and sodium salts), sorbates (alkali metal salts such as potassium salts and sodium salts), phenol, benzalkonium chloride, benzyl alcohol, chlorobutanol, and methylparaben. Examples of the antiseptics include ethanol, benzalkonium chloride, parahydroxybenzoic acid, and chlorobutanol. These additives may be included singly or in combination of two or more thereof.

Examples of the culture residues include components derived from a culture medium, contaminant proteins, and bacterial cell components.

The form of the enzyme preparation of the present invention is not particularly limited, and examples of the form include forms of liquids and solids (such as powders and granules). The composition can be prepared with a generally known method.

### 7. Intended Use

The predetermined protein-glutaminase can be used in a known use of a protein-glutaminase. For example, the predetermined protein-glutaminase can be used for the purpose of modifying a protein. Therefore, the present invention also provides a protein modifier including the predetermined protein-glutaminase.

A specific aspect of the modification of a protein is not particularly limited as long as it is a change in a property of a protein caused by generation of a carboxyl group due to deamidation of a γ-amide group and a β-amide group of a glutamine residue and an asparagine residue in the protein. Specifically, examples of the modification of a protein include an increase in the solubility of a protein, an increase in the water dispersibility, an improvement in the emulsifying power, and the emulsion stability.

The predetermined protein-glutaminase has improved thermostability, and therefore the enzyme preparation of the present invention is particularly useful in the case of treating a protein to be modified at a relatively high temperature and/or in the case of treating the protein under a heating condition for a long time. Specifically, the predetermined protein-glutaminase is preferably used in the case of treating a protein to be modified under a heating condition of 40 to 80°C, preferably 46 to 77°C, more preferably 53 to 74°C, and particularly preferably 60 to 70°C.

### 8. Method for Producing Modified Protein Material

As described above, the predetermined protein-glutaminase can be used for the purpose of modifying a protein. Therefore, the present invention also provides a method, for producing a modified protein material, including a step of making the predetermined protein-glutaminase act on a protein material.

In the method for producing of the present invention, a mixture containing a protein material and the predetermined protein-glutaminase is subjected to a condition for action of the predetermined protein-glutaminase to advance a protein-modifying reaction.

The protein material is not particularly limited as long as it is a material containing a protein, and may be edible or inedible. The edible protein material can be used as food and drink or as a material for production of food and drink. The inedible protein material can be used as a material for an experiment for a protein, a medical material, and a perfumery material.

A specific example of the protein material is a protein material obtained from a protein source through a treatment in which the protein content is increased with a known method, and is appropriately selected by those skilled in the art. Examples of the edible protein material include plant protein materials obtained from a food containing a plant protein, and animal proteins. Examples of the plant protein include bean proteins such as soybean proteins, broad bean proteins, pea proteins, chickpea proteins, mung bean proteins, and lupin proteins, grain proteins such as wheat proteins, rye proteins, oat proteins, and corn proteins, and proteins of nuts such as canary seeds, linseeds, almonds, cashew nuts, hazelnuts, pecan nuts, macadamia nuts, pistachios, walnuts, Brazil nuts, peanuts, coconuts, hemp seeds (industrial hemps), pili nuts, chestnuts, sesame, and pine nuts. Examples of the animal proteins include proteins of meat, fish, and eggs. Examples of the inedible protein material include albumin and globulin derived from a biological sample such as egg albumen and a serum.

The content of the protein contained in these protein materials is not particularly limited, and is, for example, 30 wt% or more, 40 wt% or more, or 50 wt% or more, preferably 60 wt% or more, more preferably 70 wt% or more, and still more preferably 80 wt% or more. The upper limit of the content of the protein contained in the protein material is not particularly limited, and is, for example, 95 wt% or less, 90 wt% or less, 85 wt% or less, 80 wt% or less, 70 wt% or less, or 60 wt% or less.

The content of the protein material in the mixture is an amount such that the mixture has a concentration of the protein contained in the protein material of, for example, 0.1 wt% or more or 0.3 wt% or more, preferably 0.7 wt% or more, and more preferably 1.4 wt% or more. The upper limit of the concentration of the protein contained in the protein material in the mixture is not particularly limited, and is, for example, 80 wt% or less, 60 wt% or less, 40 wt% or less, 30 wt% or less, 20 wt% or less, 15 wt% or less, 10 wt% or less, 8 wt% or less, 5 wt% or less, 3 wt% or less, or 2 wt% or less.

The amount of the predetermined protein-glutaminase used is not particularly limited, and the amount of the predetermined protein-glutaminase used with respect to 1 g of the protein contained in the protein material is, for example, 0.1 U or more, preferably 0.5 U or more, more preferably 1 U or more, still more preferably 2 U or more, even more preferably 3.5 U or more, and still even more preferably 4.5 U or more. The upper limit of the amount of the predetermined protein-glutaminase used with respect to 1 g of the protein contained in the protein material is not particularly limited, and is, for example, 45 U or less, 35 U or less, 20 U or less, 10 U or less, 8 U or less, or 5.5 U or less.

The amount of the predetermined protein-glutaminase used with respect to 1 g of the protein material is, for example, 0.1 U or more, preferably 0.5 U or more, more preferably 1 U or more, still more preferably 2 U or more, even more preferably 3 U or more, and still even more preferably 4 U or more. The upper limit of the amount of the predetermined protein-glutaminase used with respect to 1 g of the protein material is not particularly limited, and is, for example, 40 U or less, 30 U or less, 20 U or less, 10 U or less, 7 U or less, or 5 U or less.

The activity of the protein deamidase (protein-glutaminase) is defined so that when benzyloxycarbonyl-L-glutaminylglycine (Z-Gln-Gly) is used as a substrate, the amount of enzyme that liberates 1 µmol of ammonia per minute is 1 unit (1 U).

The condition for action of the predetermined protein-glutaminase is appropriately determined on the basis of the optimal temperature and the optimal pH of a protein-glutaminase to be used.

In the condition for action of the predetermined protein-glutaminase, the temperature condition is, for example, 50 to 80°C, preferably 60 to 80°C, and more preferably 63 to 80°C. The predetermined protein-glutaminase is excellent in thermostability, and therefore is particularly useful when used under a condition of 65°C or higher. From such a viewpoint, the temperature condition is more preferably 65 to 80°C, still more preferably 65 to 75°C, even more preferably 65 to 70°C, and still even more preferably 65 to 67°C. Meanwhile, under a condition of lower than 65°C, the lower the temperature is, the lower the relative value of the activity of the predetermined protein-glutaminase tends to be with respect to the activity at the optimal temperature. However, the predetermined protein-glutaminase has high protein deamidation ability, so that excellent protein deamidation ability is exhibited even under a condition of lower than 65°C. From such a viewpoint, the temperature condition is preferably, for example, 50°C or higher and lower than 65°C, 55°C or higher and lower than 65°C, 60°C or higher and lower than 65°C, or 63°C or higher and lower than 65°C.

In the condition for action of the predetermined protein-glutaminase, the pH condition is, for example, 2 to 12, preferably 3 to 10, and more preferably 4 to 9.

The time for which the predetermined protein-glutaminase is made to act is not particularly limited, and is to be appropriately determined according to the production scale or the like. For example, the time is 1 hour or more, preferably 8 hours or more, more preferably 16 hours or more, and still more preferably 20 hours or more. The upper limit of the time range is not particularly limited, and is, for example, 40 hours or less, 30 hours or less, or 25 hours or less.

After completion of the reaction, enzyme deactivation treatment is performed, followed by cooling and, as necessary, post-treatment to obtain a modified protein material.

### EXAMPLES

Hereinafter, the present invention will be specifically described with reference to Examples, but the present invention is not to be construed as being limited to the following Examples.

### [Test Example 1]

### (1) Selection of Thermostable Protein-Glutaminase-Producing Strain

From a nonpublic strain library possessed by the applicant, Chryseobacterium sp. strain 57594 and strain 61798 were selected using protein-glutaminase activity and temperature stability as indexes, and stocked in a culture solution containing glycerol.

### (2) Culture of Selected Strain

The components in Table 1 were dissolved in water at concentrations shown in Table 1, and the resulting solution was autoclaved at 121°C for 30 minutes to prepare a culture medium. A selected strain was inoculated into the culture medium and cultured at 120 rpm at 30°C for 40 to 48 hours. After the culture, the culture medium was centrifuged to collect a bacterial cell, and only the supernatant was sampled. Diatomaceous earth was added to the supernatant, the resulting product was filtered, and the culture filtrate was concentrated using an ultrafiltration membrane.

### (3) Purification of Enzymes

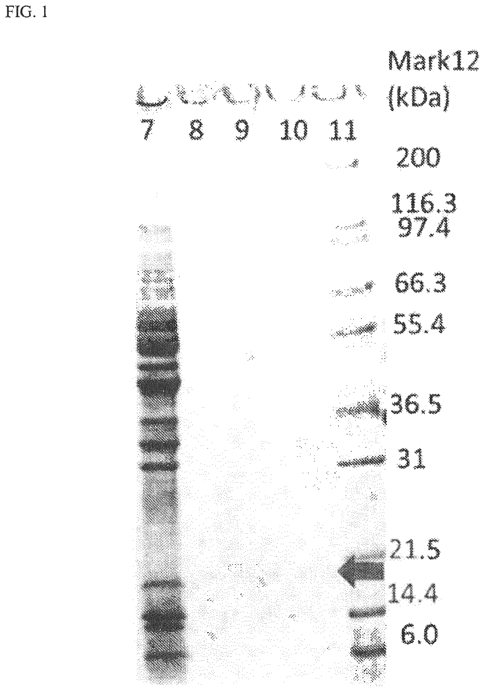

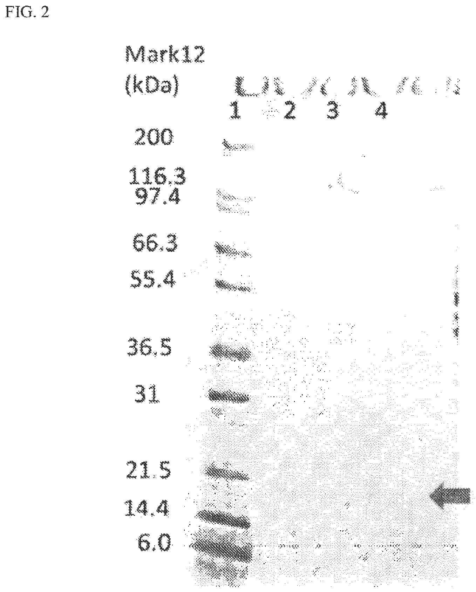

The concentrated liquid was treated by salting-out, Phenyl-sepharose, and Sephacryl S-100 to purify the enzyme. Figs. 1 and 2 show SDS-PAGE images of the concentrated fractions after the purification of the culture liquids of strain 57594 and strain 61798, respectively. In Fig. 1, the lane 7 represents the fraction before purification, the lanes 8 to 10 represent the purified fraction from the cultured strain 57594, and the lane 11 represents a molecular marker. In Fig. 2, the lane 1 represents a molecular marker, and the lanes 2 to 4 represent the purified fraction from the cultured strain 61798. As shown in Figs. 1 and 2, an 18 kDa purified fraction (band indicated by an arrow) was confirmed as a purified product.

**[Table 1]**

| Lactose-3 medium | % (w/v) |
|---|---|
| Lactose monohydrate (FUJIFILM Wako Pure Chemical Corporation) | 0.5 |
| Bacto peptone (Difco) | 2 |
| Na₂HPO₄. 12H₂O | 0.34 |
| KH₂PO₄ | 0.025 |
| MgSO₄·7H₂O | 0.025 |
| FeSO₄·7H₂O | 0.005 |
| pH7.2 | |

### (4) Enzymological Property Evaluation Test

The 18 kDa purified fraction obtained from the culture solution of each of strain 57594 and strain 61798 (protein-glutaminases derived from strain 57594 and strain 61798) was evaluated for the following enzymological properties. A protein-glutaminase derived from existing Chryseobacterium proteolyticum strain 9670 (PG for comparison) was also evaluated for the following enzymological properties in the same manner.

**[Table 2]**

| | |
|---|---|
| Example 1 | Protein-glutaminase derived from Chryseobacterium sp. strain 57594 |
| Example 2 | Protein-glutaminase derived from Chryseobacterium sp. strain 61798 |
| Comparative Example 1 | Protein-glutaminase derived from Chryseobacterium proteolyticum strain 9670 |

### (4-1) Measurement of Protein-Glutaminase Activity

- N-benzyloxycarbonyl-L-glutaminylglycine (Z-Gln-Gly; PEPTIDE INSTITUTE, INC.) was dissolved in a 0.2 mol/L phosphate buffer (pH 6.5) to prepare a 30 mmol/L solution to be used as a substrate solution.
- Into a test tube, 0.1 mL of an enzyme solution to be subjected to activity measurement was put, the test tube was allowed to stand in a constant temperature bath at 37 ± 0.5°C for 1 minute, and then 1 mL of the substrate solution allowed to stand at 37 ± 0.5°C for 10 minutes in advance was added to and immediately mixed with the enzyme solution.
- This solution was allowed to stand for 10 minutes to perform an enzyme reaction, and then 1 mL of a 0.4 mol/L trichloroacetic acid solution was added to stop the enzyme reaction.
- A measurement blank was prepared by putting 0.1 mL of the enzyme solution into a test tube, and 1 mL of a 0.4 mol/L trichloroacetic acid solution and 1 mL of the substrate solution were added in this order.
- A color development reaction was performed using Ammonia Test Wako (FUJIFILM Wako Pure Chemical Corporation) to determine the amount of ammonia liberated due to the enzyme reaction for 10 minutes on the basis of the absorbance value at a wavelength of 630 nm.
- The amount of the enzyme that produced 1 µmol of ammonia per minute was defined as 1 unit (1 U), and the activity value was calculated from the amount of ammonia liberated due to the enzyme reaction.

### (4-2) Temperature Stability Test

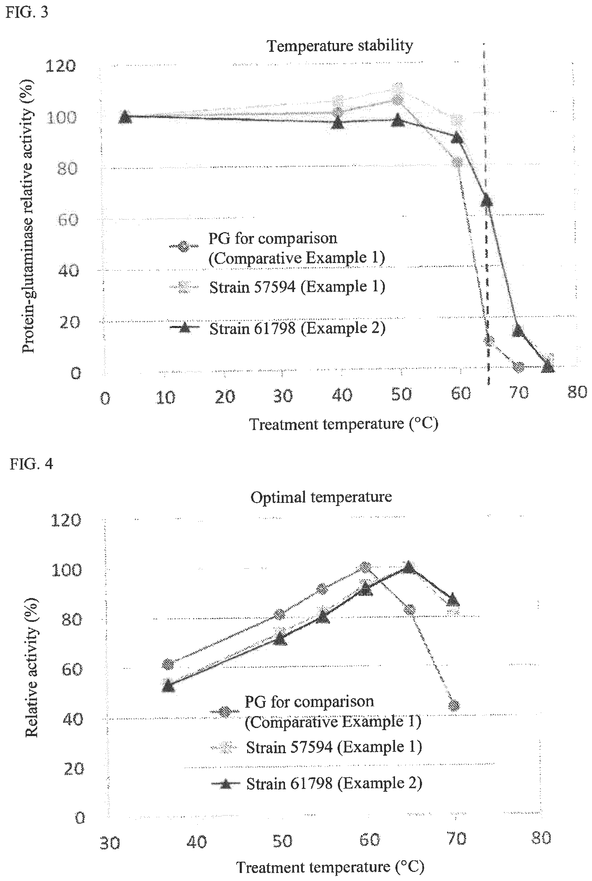

The purified fraction was substituted with a 0.2 mol/L phosphate buffer (pH 6.5), and allowed to stand at 4°C for 10 minutes or heated at 40°C, 50°C, 60°C, 65°C, 70°C, or 75°C for 10 minutes. Thereafter, an enzyme reaction was performed with the method described in (4-1), thus the protein-glutaminase activity was measured, and when the protein-glutaminase activity of the purified fraction exposed to the condition of 4°C was regarded as 100%, the relative amount of the protein-glutaminase activity of the purified fraction exposed to the heating condition at each temperature was calculated as the protein-glutaminase relative activity (%). Fig. 3 shows the results. As shown in Fig. 3, while the PG for comparison at 65°C had a relative activity of 11%, the protein-glutaminase derived from strain 57594 had a relative activity of 65%, and the protein-glutaminase derived from strain 61798 had a relative activity of 66%, resulting in a remarkable improvement in thermostability.

### (4-3) Optimum Temperature Test

The protein-glutaminase activity was measured in the same manner as in (4-1) except that the enzyme reaction temperature was changed to 37°C, 50°C, 55°C, 60°C, 65°C, or 70°C, and when the protein-glutaminase activity under a temperature condition where each purified fraction exhibited the maximum activity (60°C for the PG for comparison and 65°C for the PG derived from strain 57594 and the PG derived from strain 61798) was regarded as 100%, the relative amount of the protein-glutaminase activity of the purified fraction exposed to each temperature condition was calculated as the protein-glutaminase relative activity (%). Fig. 4 shows the results. As shown in Fig. 4, while the optimal temperature was 60°C for the PG for comparison, the optimal temperature was improved to 65°C for the protein-glutaminases derived from strain 57594 and strain 61798.

### (4-4) pH Stability Test

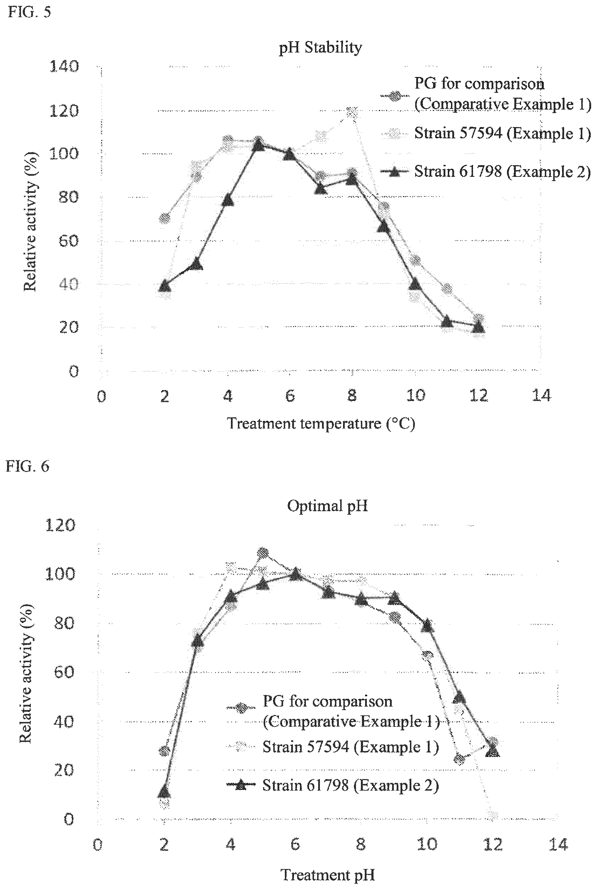

The pH of the purified fraction was adjusted to 2, 3, 4, 5, 6, 7, 8, 9, 10, 11, or 12 with Britton-Robinson buffer, and the purified fraction was allowed to stand under each pH at 37°C overnight (for 18 hours). Thereafter, an enzyme reaction was performed with the method described in (4-1), thus the protein-glutaminase activity was measured, and when the protein-glutaminase activity under a pH of 6 was regarded as 100%, the relative amount of the protein-glutaminase activity of the purified fraction exposed to each pH condition was calculated as the protein-glutaminase relative activity (%). Fig. 5 shows the results.

### (4-5) Optimal pH Test

The protein-glutaminase activity was measured in the same manner as in (4-1) except that the pH of the substrate solution was adjusted to 2, 3, 4, 5, 6, 7, 8, 9, 10, 11, or 12 with Britton-Robinson buffer and that an enzyme reaction was performed under each pH, and when the protein-glutaminase activity under a pH of 6 was regarded as 100%, the relative amount of the protein-glutaminase activity of the purified fraction exposed to each pH condition was calculated as the protein-glutaminase relative activity (%). Fig. 6 shows the results.

### (5) Sequence

A degenerate primer was designed from the sequence information of a bacterium belonging to the genus Chryseobacterium estimated to be closely related to strain 57594 and strain 61798. The degenerate primer was used for PCR with the genomic DNAs of strain 57594 and strain 61798 as templates using PrimeSTAR (registered trademark) GXL DNA Polymerase (manufactured by Takara Bio Inc.) to amplify the DNA fragments. The amplified fragments were collected with NucleoSpin (registered trademark) Gel and PCR Clean-up (manufactured by Takara Bio Inc.), and sequence analysis was performed using the degenerate primer. The sequence analysis determined the amino acid sequence of the protein-glutaminase derived from strain 57594 (SEQ ID NO: 1); the base sequence around the protein-glutaminase gene derived from strain 57594 including a structural gene (SEQ ID NO: 3) encoding SEQ ID NO: 1 (SEQ ID NO: 5); the amino acid sequence of the protein-glutaminase derived from strain 61798 (SEQ ID NO: 2); and the base sequence around the protein-glutaminase gene derived from strain 61798 including a structural gene (SEQ ID NO: 4) encoding SEQ ID NO: 2 (SEQ ID NO: 6).

### [Test Example 2]

In this Test Example, a modification (deamidation) reaction of various protein materials was performed using the protein-glutaminase derived from strain 57594 of Example 1 and the protein-glutaminase derived from strain 61798 of Example 2. The protein-glutaminase derived from existing Chryseobacterium proteolyticum strain 9670 (PG for comparison) of Comparative Example 1 was also used for performing the following modification (deamidation) reaction of various proteins.

Specifically, 20 mg of a protein material was added to 1 mL of a 50 mM phosphate buffer (pH 7.0) and suspended, a protein-glutaminase was added in an amount of 0.23 mg/g-protein (each component was added in an amount shown in Table 3 more specifically), and a reaction was performed for 24 hours under a condition of 55°C, 60°C, 65°C, or 70°C. Thereafter, 1 mL of a 0.4 M trichloroacetic acid solution was added to stop the reaction. The amount of free ammonia was determined using Ammonia Test Wako (FUJIFILM Wako Pure Chemical Corporation). The obtained amount of free ammonia was divided by the amount of free ammonia in the protein material suspension before adding the protein-glutaminase to derive the protein deamidation rate. The amount of free ammonia in the protein material suspension before adding the protein-glutaminase was measured as follows. Before adding the enzyme, 3 N concentrated sulfuric acid was added to and mixed with the protein material suspension, and the mixture was treated at 110°C for 3 hours, then cooled, neutralized with 6 N sodium hydroxide, and centrifuged to obtain a supernatant. The amount of free ammonia in the obtained supernatant was determined using Ammonia Test Wako (FUJIFILM Wako Pure Chemical Corporation). The ratio of the protein deamidation rate in each Example to the protein deamidation rate in the case of using the PG of Comparative Example 1 was derived as the deamidation acceleration rate. Table 3 shows the results.

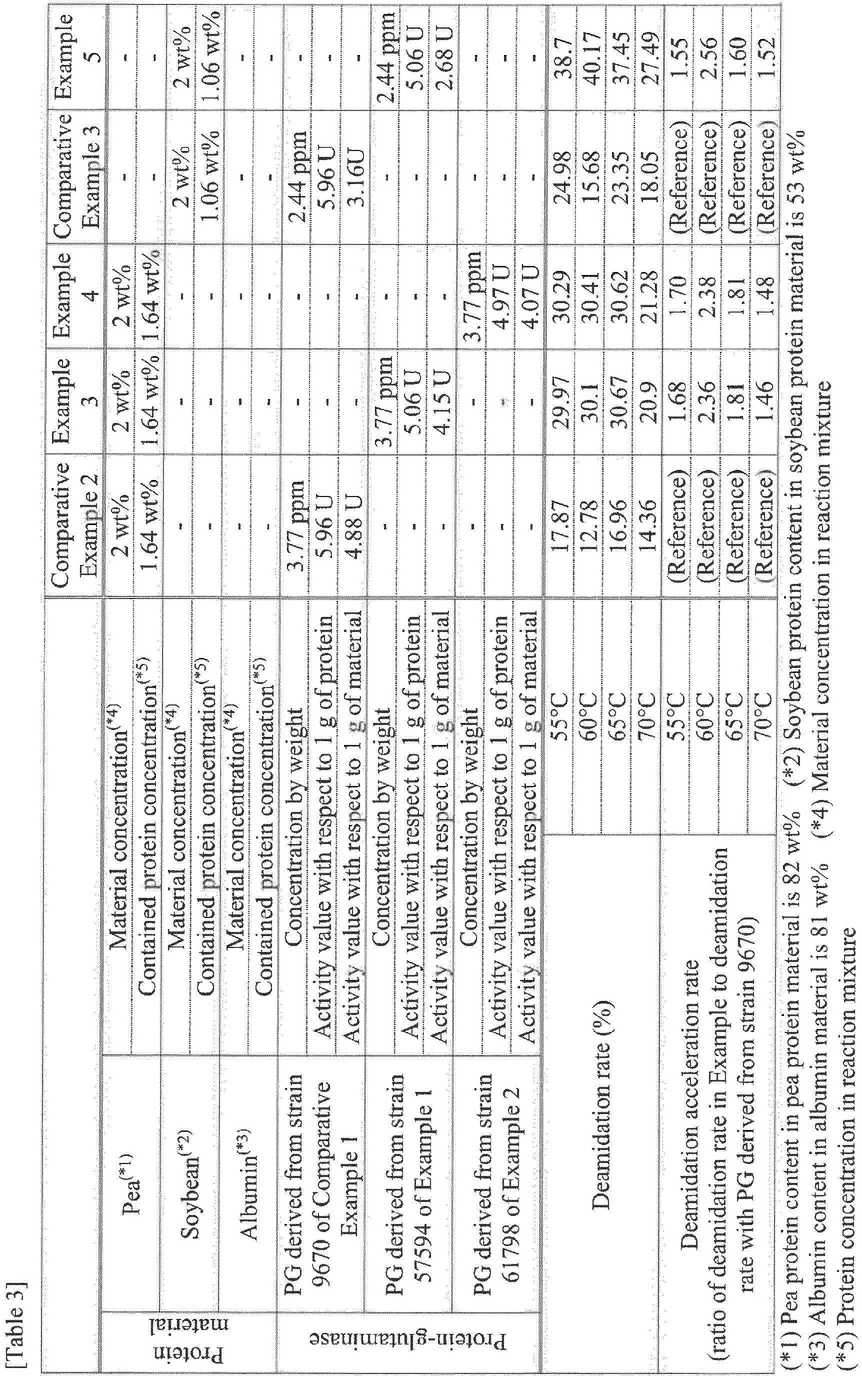

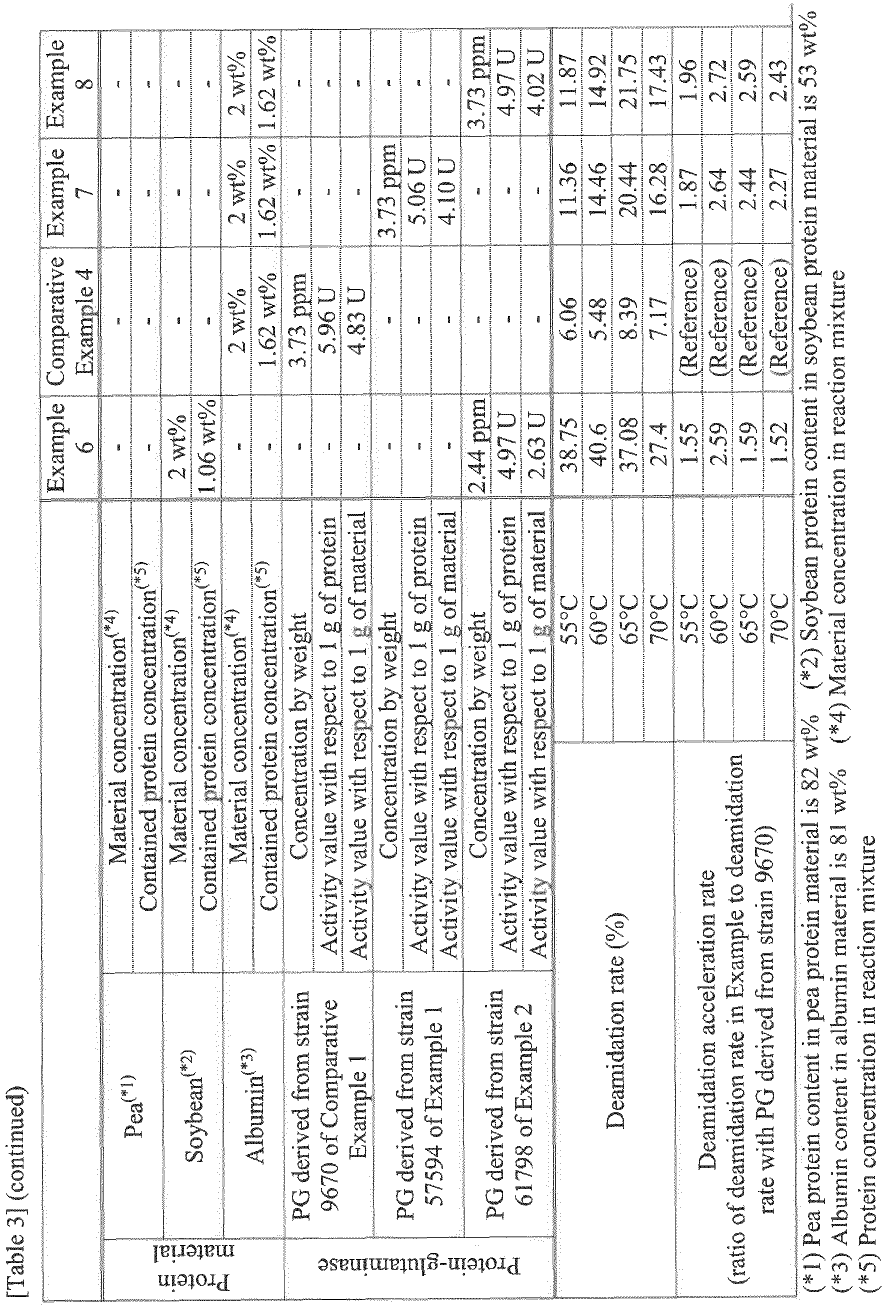

As is apparent from Table 3, for all of the food materials, a more excellent protein deamidation rate was observed in the cases of using the protein-glutaminase derived from strain 57594 of Example 1 and the protein-glutaminase derived from strain 61798 of Example 2 (Examples 3 to 8) than in the cases of using the protein-glutaminase of Comparative Example 1 (Comparative Examples 2 to 4) regardless of the reaction temperature. In particular in the cases of a food material such as a pea or a soybean, a particularly high protein deamidation acceleration rate was observed at a reaction temperature of 60°C (Examples 3 to 6), and in the cases of albumin, a high protein deamidation acceleration rate was observed at a reaction temperature of 60°C, 65°C, 70°C, and particularly 60°C (Examples 7 to 8).

### [Test Example 3]

In this Test Example, the solubilizing ability for a pea protein was tested using the protein-glutaminase derived from strain 57594 of Example 1 and the protein-glutaminase derived from strain 61798 of Example 2. The protein-glutaminase derived from existing Chryseobacterium proteolyticum strain 9670 (PG for comparison) of Comparative Example 1 was also used for the following test of the solubilizing ability in the same manner.

Specifically, 100 mg of a pea protein material (protein content: 82 wt%) was added to 10 mL of a 50 mM phosphate buffer (pH 7.0) and suspended, a protein-glutaminase was added in an amount of 0.23 mg/g-protein (each component was added in an amount shown in Table 4 more specifically), and a reaction was performed for 24 hours under a condition of 65°C.

A sample was taken in an amount of 1 mL, and 1 mL of a 0.4 M trichloroacetic acid solution was added to stop the reaction. The amount of free ammonia was determined using Ammonia Test Wako (FUJIFILM Wako Pure Chemical Corporation). The obtained amount of free ammonia was divided by the amount of free ammonia in the protein material suspension before adding the protein-glutaminase to derive the protein deamidation rate. Table 4 shows the results.

In the remaining sample, the reaction was stopped by immersing the container containing the reaction composition in boiling water to perform a heat treatment. Salt removal was performed with purified water, and freeze-drying was performed to obtain a deamidated protein (modified protein) powder. In 1 mL of Britton-Robinson buffer having a pH of 2 to 12, 2 mg of the obtained freeze-dried deamidated protein (modified protein) powder was dissolved, and the resulting solution was shaken at room temperature for 30 minutes. The solution was centrifuged at 15,000 × g for 5 minutes, and the concentration of the dissolved protein in the supernatant was measured with the Lowry method. The ratio of the concentration of the dissolved protein in each Example to the concentration of the dissolved protein in the case of using the PG of Comparative Example 1 was derived as the protein dissolution acceleration rate. Table 4 shows the results (Table 4 has no data in the portions with a diagonal line).

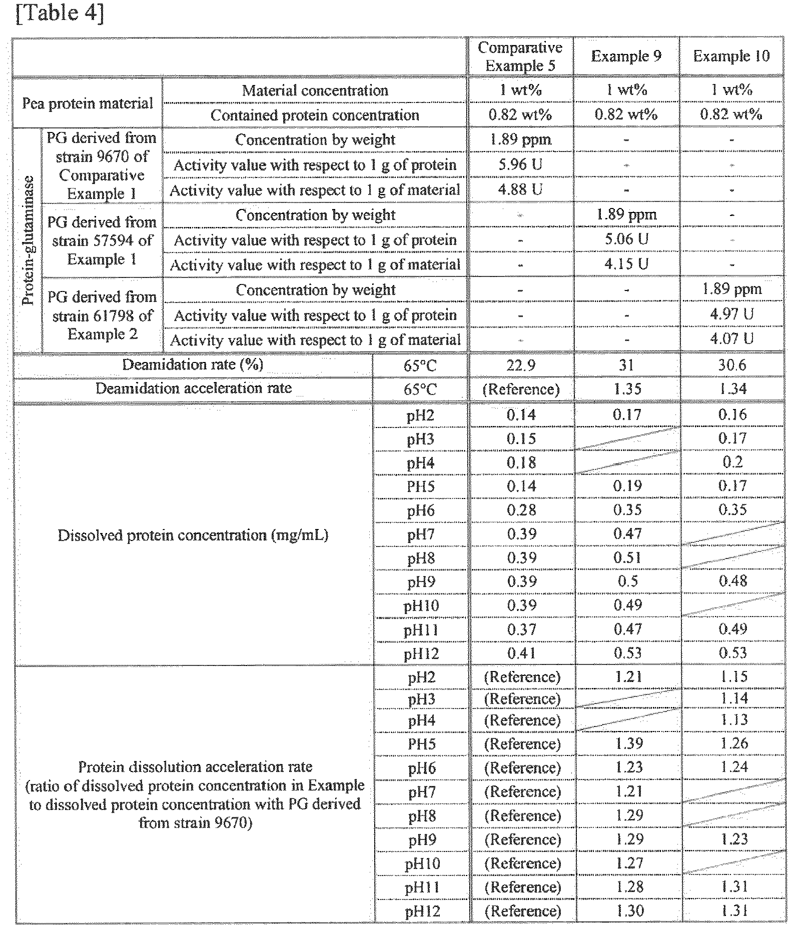

As is apparent from Table 4, a more excellent protein deamidation rate was observed in the cases of using the protein-glutaminase derived from strain 57594 of Example 1 and the protein-glutaminase derived from strain 61798 of Example 2 (Examples 9 to 10) than in the case of using the protein-glutaminase of Comparative Example 1 (Comparative Example 5). As compared with the case of using the protein-glutaminase of Comparative Example 1 (Comparative Example 5), the solubility of the obtained modified protein was improved at any pH (Examples 9 to 10), and a correlation was observed between the improvement in the solubility and the improvement in the protein deamidation rate.

### [Test Example 4]

In this Test Example, the deamidation ability for egg albumin under an alkaline condition was tested using the protein-glutaminase derived from strain 57594 of Example 1 and the protein-glutaminase derived from strain 61798 of Example 2. The protein-glutaminase derived from existing Chryseobacterium proteolyticum strain 9670 (PG for comparison) of Comparative Example 1 was also used for the following test of the deamidation ability in the same manner.

Specifically, 20 mg of egg albumin (protein content: 81 wt%) was added to 1 mL of Britton-Robinson buffer (pH 7.0 or pH 10.0) and suspended, a protein-glutaminase was added in an amount of 0.23 mg/g-protein (each component was added in an amount shown in Table 5 more specifically), and a reaction was performed for 24 hours at 37°C. Thereafter, 1 mL of a 0.4 M trichloroacetic acid solution was added to stop the reaction. The amount of free ammonia was determined using Ammonia Test Wako (FUJIFILM Wako Pure Chemical Corporation). The obtained amount of free ammonia was divided by the amount of free ammonia in the protein material suspension before adding the protein-glutaminase to derive the protein deamidation rate. The ratio of the protein deamidation rate at a pH of 10 to the protein deamidation rate in the case of a treatment at a pH of 7 was derived as the deamidation acceleration rate under an alkaline condition. Table 5 shows the results.

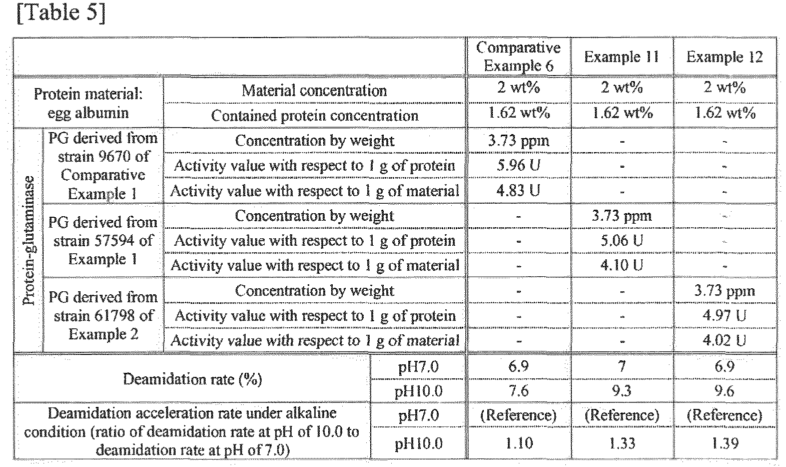

As is apparent from Table 5, an improvement in the protein deamidation acceleration rate under an alkaline condition was observed in the cases of using the protein-glutaminase derived from strain 57594 of Example 1 and the protein-glutaminase derived from strain 61798 of Example 2 (Examples 11 to 12) as compared with the case of using the protein-glutaminase of Comparative Example 1 (Comparative Example 6).

## Claims

1. A protein-glutaminase comprising a polypeptide shown in any one of the following (1) to (3):
(1) a polypeptide having an amino acid sequence shown in SEQ ID NO: 1 or 2;
(2) a polypeptide having an amino acid sequence obtained by substituting, adding, inserting, or deleting one or more amino acid residues in an amino acid sequence shown in SEQ ID NO: 1 or 2, the polypeptide having thermostability equivalent to thermostability of a polypeptide having an amino acid sequence shown in SEQ ID NO: 1 or 2; and
(3) a polypeptide having an amino acid sequence having a sequence identity to an amino acid sequence shown in SEQ ID NO: 1 or 2 of 76% or more, the polypeptide having thermostability equivalent to thermostability of a polypeptide having an amino acid sequence shown in SEQ ID NO: 1 or 2.

2. A DNA encoding the protein-glutaminase according to claim 1.

3. An expression cassette or a recombinant vector comprising the DNA according to claim 2.

4. A transformant obtained by transforming a host with the expression cassette or the recombinant vector according to claim 3.

5. A method for producing the protein-glutaminase according to claim 1, the method comprising a step of culturing the transformant according to claim 4.

6. An enzyme preparation comprising the protein-glutaminase according to claim 1.

7. A protein modifier comprising the protein-glutaminase according to claim 1.

8. A method for producing a modified protein material, the method comprising a step of making the protein-glutaminase according to claim 1 act on a protein material.

9. The method according to claim 8, wherein the protein material is edible.
